# EUROPEAN PATENT SPECIFICATION

(11) **EP 1 333 100 B1**
(45) Date of publication and mention of the grant of the patent: **14.12.2005**
(21) Application number: 02001681.2
(22) Date of filing: 24.01.2002
(51) Int. Cl.: C12Q 1/68, B01J 19/00

(54) **RET oligonucleotide microchip for detecting hereditary cancer**
RET, Oligonukleotidmikrochip, zum, Nachweis, von, erblichem, Krebs
RET micropuce à oligonucléotides de détection de cancer héréditaire

(30) Priority: 19.11.2001 KR 2001071774
(43) Date of publication of application: 06.08.2003
(73) Proprietor: National Cancer Center, Goyang-si, Gyeonggi-do 411-764 (KR)
(72) Inventor: Park, Jae-Gahb, Seoul 137-060 (KR); Kim, Il-Jin, Seoul 138-180 (KR); Kang, Hio Chung, Seoul 158-095 (KR); Park, Jae-Hyun, Seoul 143-873 (KR)
(74) Representative: Goddar, Heinz J.

(56) References cited:
- JHIANG SISSY M: "The RET proto-oncogene in human cancers" ONCOGENE, vol. 19, no. 49, 20 November 2000 (2000-11-20), pages 5590-5597, XP002258258 ISSN: 0950-9232
- PASINI B ET AL: "RET mutations in human disease" TRENDS IN GENETICS, ELSEVIER SCIENCE PUBLISHERS B.V. AMSTERDAM, NL, vol. 12, no. 4, 1 April 1996 (1996-04-01), pages 138-144, XP004037254 ISSN: 0168-9525
- LANGMANN THOMAS ET AL: "Multiplex analysis of mutations in the RET-gene using suspension arrays" CYTOMETRY SUPPLEMENT, no. 10, 2000, pages 39-40, XP009019458 The XX Congress of the International Society for Analytical Cytology;Montpellier, France; May 20-25, 2000 ISSN: 1046-7386
- KIM IL-JIN ET AL: "RET oligonucleotide microarray for the detection of RET mutations in multiple endocrine neoplasia type 2 syndromes." CLINICAL CANCER RESEARCH: AN OFFICIAL JOURNAL OF THE AMERICAN ASSOCIATION FOR CANCER RESEARCH. UNITED STATES FEB 2002, vol. 8, no. 2, February 2002 (2002-02), pages 457-463, XP001155716 ISSN: 1078-0432

### SEQUENCING LISTINGS

<110> NATIONAL CANCER CENTER
<120> RET OLIGONUCLEOTIDE MICROCHIP AND METHOD FOR DETECTING HEREDITARY CANCER EMPLOYING SAME
<130> PCA11254/PJG/EP
<160> 67
<170> KopatentIn 1.71
<210> 1
   <211> 21
   <212> DNA
   <213> Artificial Sequence
<220>
   <223> 10-PC
<400> 1
   ggggattaaa gctggctatg g 21
<210> 2
   <211> 21
   <212> DNA
   <213> Artificial Sequence
<220>
   <223> 609M-(R)
<400> 2
   ctatggcacc cgcaactgct t 21
<210> 3
   <211> 21
   <212> DNA
   <213> Artificial Sequence
<220>
   <223> 609M-(S)
<400> 3
   ctatggcacc agcaactgct t 21
<210> 4
   <211> 16
   <212> DNA
   <213> Artificial Sequence
<220>
   <223> 609M-(G)
<400> 4
   tggcaccggc aactgc 16
<210> 5
   <211> 22
   <212> DNA
   <213> Artificial Sequence
<220>
   <223> 609M-(F)
<400> 5
   ctatggcacc ttcaactgct tc 22
<210> 6
   <211> 22
   <212> DNA
   <213> Artificial Sequence
<220>
   <223> 609M-(Y)
<400> 6
   ctatggcacc tacaactgct tc 22
<210> 7
   <211> 21
   <212> DNA
   <213> Artificial Sequence
<220>
   <223> 609M-(S)
<400> 7
   tatggcacct ccaactgctt c 21
<210> 8
   <211> 21
   <212> DNA
   <213> Artificial Sequence
<220>
   <223> 609M-(W)
<400> 8
   tatggcacct ggaactgctt c 21
<210> 9
   <211> 21
   <212> DNA
   <213> Artificial Sequence
   <220>
   <223> 609M-(C)
<400> 9
   tatggcacct gcaactgctt c 21
<210> 10
   <211> 20
   <212> DNA
   <213> Artificial Sequence
<220>
   <223> 611M-(R)
<400> 10
   acctgcaacc gcttccctga 20
<210> 11
   <211> 21
   <212> DNA
   <213> Artificial Sequence
<220>
   <223> 611M-(S)
<400> 11
   cacctgcaac agcttccctg a 21
<210> 12
   <211> 20
   <212> DNA
   <213> Artificial Sequence
<220>
   <223> 611M-(G)
<400> 12
   acctgcaacg gcttccctga 20
<210> 13
   <211> 21
   <212> DNA
   <213> Artificial Sequence
<220>
   <223> 611M-(F)
<400> 13
   acctgcaact tcttccctga g 21
<210> 14
   <211> 21
   <212> DNA
   <213> Artificial Sequence
<220>
   <223> 611M-(Y)
<400> 14
   acctgcaact acttccctga g 21
<210> 15
   <211> 21
   <212> DNA
   <213> Artificial Sequence
<220>
   <223> 611M-(S)
<400> 15
   acctgcaact ccttccctga g 21
<210> 16
   <211> 21
   <212> DNA
   <213> Artificial Sequence
<220>
   <223> 611M-(W)
<400> 16
   acctgcaact ggttccctga g 21
<210> 17
   <211> 21
   <212> DNA
   <213> Artificial Sequence
<220>
   <223> 611W-(C)
<400> 17
   acctgcaact gcttccctga g 21
<210> 18
   <211> 20
   <212> DNA
   <213> Artificial Sequence
<220>
   <223> 618M-(R)
<400> 18
   aggagaagcg cttctgcgag 20
<210> 19
   <211> 21
   <212> DNA
   <213> Artificial Sequence
<220>
   <223> 618M-(S)
<400> 19
   gaggagaaga gcttctgcga g 21
<210> 20
   <211> 16
   <212> DNA
   <213> Artificial Sequence
<220>
   <223> 618M-(G)
<400> 20
   gagaagggct tctgcg 16
<210> 21
   <211> 20
   <212> DNA
   <213> Artificial Sequence
<220>
   <223> 618M-(F)
<400> 21
   aggagaagtt cttctgcgag 20
<210> 22
   <211> 20
   <212> DNA
   <213> Artificial Sequence
<220>
   <223> 618M-(Y)
<400> 22
   aggagaagta cttctgcgag 20
<210> 23
   <211> 20
   <212> DNA
   <213> Artificial Sequence
<220>
   <223> 618M-(S)
<400> 23
   aggagaagtc cttctgcgag 20
<210> 24
   <211> 20
   <212> DNA
   <213> Artificial Sequence
<220>
   <223> 618M-(W)
<400> 24
   aggagaagtg gttctgcgag 20
<210> 25
   <211> 21
   <212> DNA
   <213> Artificial Sequence
<220>
   <223> 618W-(C)
<400> 25
   gaggagaagt gcttctgcga g 21
<210> 26
   <211 > 20
   <212> DNA
   <213> Artificial Sequence
<220>
   <223> 620M-(R)
<400> 26
   aagtgcttcc gcgagcccga 20
<210> 27
   <211> 20
   <212> DNA
   <213> Artificial Sequence
<220>
   <223> 620M-(S)
<400> 27
   aagtgcttca gcgagcccga 20
<210> 28
   <211> 20
   <212> DNA
   <213> Artificial Sequence
   <220>
   <223> 620M-(G)
<400> 28
   aagtgcttcg gcgagcccga 20
<210> 29
   <211> 20
   <212> DNA
   <213> Artificial Sequence
<220>
   <223> 620M-(F)
<400> 29
   aagtgcttct tcgagcccga 20
<210> 30
   <211> 20
   <212> DNA
   <213> Artificial Sequence
<220>
   <223> 620M-(Y)
<400> 30
   aagtgcttct acgagcccga 20
<210> 31
   <211> 20
   <212> DNA
   <213> Artificial Sequence
<220>
   <223> 620M-(S)
<400> 31
   aagtgcttct ccgagcccga 20
<210> 32
   <211> 20
   <212> DNA
   <213> Artificial Sequence
<220>
   <223> 620M-(W)
<400> 32
   aagtgcttct gggagcccga 20
<210> 33
   <211> 20
   <212> DNA
   <213> Artificial Sequence
<220>
   <223> 620W-(C)
<400> 33
   aagtgcttct gcgagcccga 20
<210> 34
   <211> 20
   <212> DNA
   <213> Artificial Sequence
<220>
   <223> 630M-(M)
<400> 34
   gatccactgc gcgacgagct 20
<210> 35
   <211> 20
   <212> DNA
   <213> Artificial Sequence
<220>
   <223> 630M-(S)
<400> 35
   gatccactga gcgacgagct 20
<210> 36
   <211> 20
   <212> DNA
   <213> Artificial Sequence
<220>
   <223> 630M-(G)
<400> 36
   gatccactgg gcgacgagct 20
<210> 37
   <211> 20
   <212> DNA
   <213> Artificial Sequence
<220>
   <223> 630M-(F)
<400> 37
   gatccactgt tcgacgagct 20
<210> 38
   <211> 20
   <212> DNA
   <213> Artificial Sequence
<220>
   <223> 630M-(Y)
<400> 38
   gatccactgt acgacgagct 20
<210> 39
   <211> 20
   <212> DNA
   <213> Artificial Sequence
<220>
   <223> 630M-(S)
<400> 39
   gatccactgt ccgacgagct 20
<210> 40
   <211> 20
   <212> DNA
   <213> Artificial Sequence
<220>
   <223> 630M-(W)
<400> 40
   gatccactgt gggacgagct 20
<210> 41
   <211> 20
   <212> DNA
   <213> Artificial Sequence
<220>
   <223> 630W-(C)
<400> 41
   gatccactgt gcgacgagct 20
<210> 42
   <211> 20
   <212> DNA
   <213> Artificial Sequence
<220>
   <223> 634M-(R)
<400> 42
   gacgagctgc gccgcacggt 20
<210> 43
   <211> 20
   <212> DNA
   <213> Artificial Sequence
<220>
   <223> 634M-(S)
<400> 43
   gacgagctga gccgcacggt 20
<210> 44
   <211> 16
   <212> DNA
   <213> Artificial Sequence
<220>
   <223> 634M-(G)
<400> 44
   cgagctgggc cgcacg 16
<210> 45
   <211> 20
   <212> DNA
   <213> Artificial Sequence
<220>
   <223> 634M-(F)
<400> 45
   gacgagctgt tccgcacggt 20
<210> 46
   <211> 20
   <212> DNA
   <213> Artificial Sequence
<220>
   <223> 634M-(Y)
<400> 46
   gacgagctgt accgcacggt 20
<210> 47
   <211> 20
   <212> DNA
   <213> Artificial Sequence
<220>
   <223> 634M-(S)
<400> 47
   gacgagctgt cccgcacggt 20
<210> 48
   <211> 20
   <212> DNA
   <213> Artificial Sequence
<220>
   <223> 634M-(W)
<400> 48
   gacgagctgt ggcgcacggt 20
<210> 49
   <211> 20
   <212> DNA
   <213> Artificial Sequence
<220>
   <223> 634W-(C)
<400> 49
   gacgagctgt gccgcacggt 20
<210> 50
   <211> 20
   <212> DNA
   <213> Artificial Sequence
   <220>
   <223> 11PC
<400> 50
   ccgctgtcct cttctccttc 20
<210> 51
   <211> 20
   <212> DNA
   <213> Artificial Sequence
<220>
   <223> 768M-(Q)
<400> 51
   tccccgagtc agcttcgaga 20
<210> 52
   <211> 21
   <212> DNA
   <213> Artificial Sequence
<220>
   <223> 768M-(K)
<400> 52
   ctccccgagt aagcttcgag a 21
<210> 53
   <211> 20
   <212> DNA
   <213> Artificial Sequence
<220>
   <223> 768M-(A)
<400> 53
   tccccgagtg cgcttcgaga 20
<210> 54
   <211> 20
   <212> DNA
   <213> Artificial Sequence
<220>
   <223> 768M-(G)
<400> 54
   tccccgagtg ggcttcgaga 20
<210> 55
   <211> 20
   <212> DNA
   <213> Artificial Sequence
<220>
   <223> 768M-(V)
<400> 55
   tccccgagtg tgcttcgaga 20
<210> 56
   <211> 20
   <212> DNA
   <213> Artificial Sequence
<220>
   <223> 768M-(D)
<400> 56
   tccccgagtg accttcgaga 20
<210> 57
   <211> 21
   <212> DNA
   <213> Artificial Sequence
<220>
   <223> 768M-(D)
<400> 57
   tccccgagtg atcttcgaga c 21
<210> 58
   <211> 20
   <212> DNA
   <213> Artificial Sequence
<220>
   <223> 768W-(E)
<400> 58
   tccccgagtg agcttcgaga 20
<210> 59
   <211> 20
   <212> DNA
   <213> Artificial Sequence
<220>
   <223> 804M-(L)
<400> 59
   ctcctcatcc tggagtacgc 20
<210> 60
   <211> 21
   <212> DNA
   <213> Artificial Sequence
<220>
   <223> 804M-(M)
<400> 60
   cctcctcatc atggagtacg c 21
<210> 61
   <211> 21
   <212> DNA
   <213> Artificial Sequence
<220>
   <223> 804M-(L)
<400> 61
   cctcctcatc ttggagtacg c 21
<210> 62
   <211> 20
   <212> DNA
   <213> Artificial Sequence
<220>
   <223> 804M-(E)
<400> 62
   ctcctcatcg aggagtacgc 20
<210> 63
   <211> 20
   <212> DNA
   <213> Artificial Sequence
<220>
   <223> 804M-(A)
<400> 63
   ctcctcatcg cggagtacgc 20
<210> 64
   <211> 20
   <212> DNA
   <213> Artificial Sequence
<220>
   <223> 804M-(G)
<400> 64
   ctcctcatcg gggagtacgc 20
<210> 65
   <211> 20
   <212> DNA
   <213> Artificial Sequence
<220>
   <223> 804W-(V)
<400> 65
   ctcctcatcg tggagtacgc 20
<210> 66
   <211> 24
   <212> DNA
   <213> Artificial Sequence
<220>
   <223> 918M-(T)
<400> 66
   cagttaaatg gacggcaatt gaat 24
<210> 67
   <211> 24
   <212> DNA
   <213> Artificial Sequence
<220>
   <223> 918W-(M)
<400> 67
   cagttaaatg gatggcaatt gaat 24
<210> 68
   <211> 28
   <212> DNA
   <213> Artificial Sequence
<220>
   <223> Exon-10F
<400> 68
   gtaggaattc gacacgagcc tggggagc 28
<210> 69
   <211> 30
   <212> DNA
   <213> Artificial Sequence
   <220>
   <223> Exon-10R
<400> 69
   catcgaattc ttgttggacc tcagatgtgc 30
<210> 70
   <211> 30
   <212> DNA
   <213> Artificial Sequence
<220>
   <223> Exon-11F
<400> 70
   gtaggaattc gcagcctgta cccagtggtg 30
<210> 71
   <211> 29
   <212> DNA
   <213> Artificial Sequence
<220>
   <223> Exon-11R
<400> 71
   catcgaattc accggaagag gagtagctg 29
<210> 72
   <211> 39
   <212> DNA
   <213> Artificial Sequence
<220>
   <223> Exon-13F
<400> 72
   taatacgact cactataggg cttccaggag cgatcgttt 39
<210> 73
   <211> 36
   <212> DNA
   <213> Artificial Sequence
<220>
   <223> Exon-13R
<400> 73
   atttaggtga cactatagca ccctgcagct ggcctt 36
<210> 74
   <211> 19
   <212> DNA
   <213> Artificial Sequence
<220>
   <223> Exon-14F
<400> 74
   tggctcctgg aagacccaa 19
<210> 75
   <211> 19
   <212> DNA
   <213> Artificial Sequence
<220>
   <223> Exon-14R
<400> 75
   tgcacgcacc ttcatctcg 19
<210> 76
   <211> 43
   <212> DNA
   <213> Artificial Sequence
<220>
   <223> Exon-16F
<400> 76
   taatacgact cactataggg agagttagag taacttcaat gtc 43
<210> 77
   <211> 37
   <212> DNA
   <213> Artificial Sequence
<220>
   <223> Exon-16R
<400> 77
   atttaggtga cactatagta acctccaccc caagaga 37

## Description

### Field of the Invention

The present invention relates to an RET oligonucleotide microchip for detecting mutation in the mutational hot spot regions of RET gene, a manufacturing process thereof and a method for detecting hereditary cancer employing same.

### Background of the Invention

It has been estimated that about 5~10% of cancer is hereditary in nature, and such hereditary cancer is caused by gene mutation. Accordingly, by detecting gene mutation in family members of hereditary cancer patient, hereditary cancer can be prevented or cured at an early stage. Studies of hereditary cancer also provide key clues to unravel tumorigenesis as well as means for the prevention and early diagnosis of cancer. Such studies have already identified several kinds of tumor suppressor genes.

To test germline mutation for members of high-risk families, an oligonucleotide microchip (hereinafter, referred to as "oligo chip") has been developed. It is used to examine the presence of germline mutation in high-risk family members, detect the hereditary cancer in its early stage and provides a regular medical treatment to the member having germline mutation. The result of such tests also relieves family members having no germline mutation of the fear of cancer.

Generally, used in a technique involving hybridization of a test oligonucleotide with oligonucleotides fixed on the surface of a solid matrix, i.e. a glass slide or gel. Such oligonucleotide-coated solid matrix may be prepared in two ways: by directly synthesizing oligonucleotides on the solid matrix surface, or by fixing pre-synthesized oligonucleotides on the solid matrix surface. The former uses a photolithographic oligonucleotide synthesizing technique: A surface bearing photoprotected hydroxyls is illuminated through a photolithographic mask to generate free hydroxyl groups in the photodeprotected regions, and then, the hydroxyl groups are coupled to deoxynucleoside phosphoramidite (Pease, A. C. et al., *Proc. Natl.* *Acad. Sci. USA* 91:5022~5026, 1994).

In the latter method, oligonucleotides carrying homopolymer tails with terminal deoxyribonucleotidyl transferase are spotted onto a nylon membrane and covalently bound by UV irradiation (Saiki, R. K. et al., *Proc. Natl. Acad. Sci. USA* 86:6230~6234, 1989). Also reported is an improved method based on the formation of amide bonds between carboxyl groups of the membranes and amino-linkers attached to the 5' end of oligonucleotides, to avoid non-specific fixation that occurs when heat or ultraviolet light is used (Zhang, Y. et al., *Nucleic Acids Res.* 19:3929~3933, 1991).

The method of hybridizing a radioisotope labeled or non-labeled target DNA with such a DNA chip prepared as above has been successfully applied for detecting RAS point mutation, cystic fibrosis deletion and various point mutation as well as DNA sequencing (Cantor, C. R. et al., *Genomics* 13:1378~383, 1992). It has also been used in detecting gene mutation and in the study of gene polymorphism (Lipshutz, R. J. et al., *Biotechniques* 19:442~447, 1995). Further, this technique is expected to provide a powerful tool for efficient determination of neoplasias and HLA genotyping in addition to diagnosis of hereditary diseases (Saiki, R. K. et al., *Proc. Natl. Acad. Sci. USA* 86:6230~6234, 1989).

However, oligo chips for the detection of gene mutation are not easy to manufacture, and therefore, only a few oligo chips have been commercialized, e.g., by Affymetrix (Santa Clara, CA 95051), and their applicability is limited to detect gene mutation in only a few genes, e. g., p53, ATM and BRCA1. The oligo chip sold by Affymetrix is manufactured by directly synthesizing oligonucleotides on the surface of a solid matrix using phothlithography. However, this oligo chip has many problems, e. g., its production cost is high; it requires the use of expensive equipments; it is not possible to fix purified oligonucleotides of good quality on the solid matrix surface; and the amount of hybridized product generated on the solid matrix surface is insufficient for quantitative analysis.

On the other hand, multiple endocrine neoplasia type 2 (MEN2) syndromes are inherited in an autosomal dominant fashion with high penetrance. There are three subtypes, namely, MEN2A, MEN2B and FMTC (familial medullary thyroid carcinoma), and mutation in the RET gene plays an important role in the MEN2 syndromes: missense mutation at one of 9 codons (codons 609, 611, 618, 620, 630, 634, 768, 804, and 918) of RET gene is observed in more than 95% MEN2 cases. To examine the presence of mutation in these mutational hot spot regions of RET gene, many tools such as SSCP (single strand conformation polymorphism), PTT (protein truncation test), and gel-based sequencing have been used. However, as these tools are relatively time-consuming and labor intensive, there has been a need to develop an improved method.

The present inventors have therefore endeavored to meet the above need, and developed an RET oligo chip which is manufactured by fixing oligonucleotides on the surface of a solid matrix using an automatic microarrayer, the oligonucleotides being designed to detect missense mutation at mutational hot spot regions of RET gene. The RET oligo chip of the present invention can also be used in studies to unravel the signal transduction mechanism and tumorigenesis related to RET gene.

### Summary of the Invention

Accordingly, an object of the present invention is to provide an RET oligo chip which can be used as a fast and reliable genetic diagnostic device for detecting RET gene mutations and hereditary cancer.

In accordance with one aspect of the present invention, there is provided an RET oligonucleotide microchip as specified in claim 1 for detecting RET mutations comprising a plurality of oligonucleotides fixed on the surface of a solid matrix, wherein the oligonucleotides are designed to detect missense mutation in the mutational hot spots of RET gene.

In accordance with still another aspect of the present disclosure, there is provided a method for detecting hereditary cancer employing same.

### Brief Description of the Drawings

The above and other objects and features of the present invention will become apparent from the following description of the invention, when taken in conjunction with the accompanying drawings which respectively show;
Fig. 1 : an exterior view of the inventive RET oligo chip;
Fig. 2 : an example of the inventive RET oligo chip that has a total of 268 oligonucleotide spots printed on the surface of a 3.7 cm × 7.6 cm glass slide;
Fig. 3 : a SigmaPlot for determining the threshold value by analyzing signal intensities from each oligonucleotide spots after hybridization reaction, wherein the solid line indicates the threshold value and the dash-line indicates the mean value of the background;
Fig. 4 : the presence of RET gene mutation in each codon examined by using the inventive RET oligo chip,
a: hybridization at exon 10 (codons 609, 611, 618, and 620) and exon 11 (codons 630 and 634)
b: hybridization at exon 13 (codon 768) and exon 14 (codon 804)

### Detailed Description of the Invention

The present invention provides an RET oligonucleotide microchip for detecting RET mutations as specified in claim 1, which comprises oligonucleotides fixed on the surface of a solid matrix using an automatic microarrayer, wherein the oligonucleotides being capable of detecting missense mutation at mutational hot spot regions of RET gene.

First, the oligonucleotides are designed to detect all generable missense mutation types at 8 codons (codons 609, 611, 618, 630, 634, 768, and 804) and one missense mutation type at codon 918.

As stated above, RET gene is responsible for MEN2 syndromes, which are inherited in an autosomal dominant fashion with high penetrance and diverse clinical manifestations. The predominant RET mutation is missense mutation which is restricted to 9 codons (codons 609, 611, 618, 620, 630, 634, 768, 804 and 918). The MEN2 syndromes have 3 subtypes: multiple endocrine neoplasia type 2A (MEN2A), MEN2B, and familial medullary thyroid carcinoma (FMTC). Missense mutations at exon 10 (codons 609, 611, 618, and 620) and exon 11 (codons 630 and 634) have been identified in 98% of MEN2A families and in 85% of FMTC families. Missense mutations at codons 768 and 804 have been known to be responsible for 5~10% of FMTC cases. In addition, missense mutations at exon 16 (codon 918) have been found in 95% of MEN2B cases.

The present invention provides various oligonucleotides which can be used to detect missense mutation at the above mentioned hot spots of RET gene, which occur at a frequency of more than 95% of all cases examined. Therefore, the RET oligo chip of the present invention makes it possible to detect mutation at a confidence level of over 95%. In addition, since the oligonucleotides used in the inventive RET oligo chip is designed to detect all possible missense mutations at 8 codons, it is possible to detect any missense mutation at these codons which have not yet been discovered. At codon 918, mutation occurs predominantly as Met→ Thr type mutation, and accordingly, the oligonucleotide used in this invention is designed to detect this mutation type. Namely, as the inventive oligonucleotides are specifically designed to detect mutation at the hot spot of RET gene taking the gene characteristics into consideration, the inventive RET oligo chip provides improved accuracy and efficiency in detecting RET gene mutation.

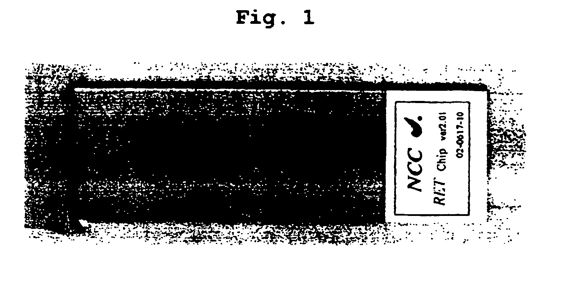

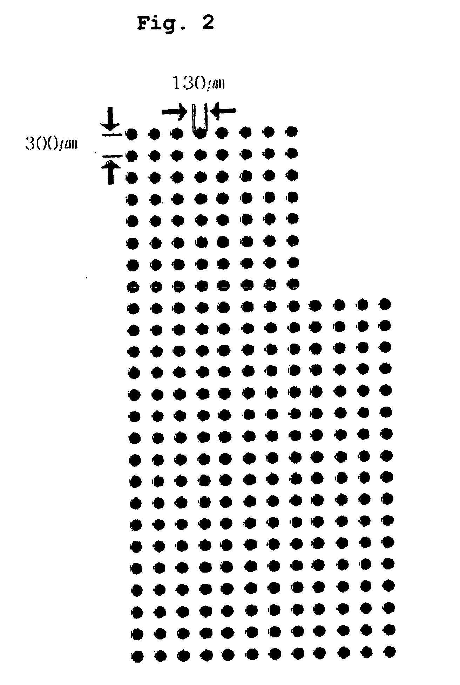

According to one aspect of the present invention, the inventive RET oligo chip has 67 types of oligonucleotides spotted and fixed on the surface of a solid matrix, wherein the oligonucleotides can detect various missense mutations at the 9 hot spot codons of RET gene. Each oligonucleotide is spotted 4 times horizontally for increased accuracy of measured signals, as shown in Table 1, Figs. 1 and 2. Positions 1 to 25 are spotted first, then positions 26 to 50, and finally, positions 51 to 67. Positions 1 and 50 are positive controls for exon 10 and 11, respectively. The other oligonucleotides are: 2-33 for exon 10 (codons 609, 611, 618, and 620); 34~49 for exon 11 (codons 630 and 634); 51~58 for exon 13 (codon 768); 59~65 for exon 14 (codon 804); and 66~67 for exon 16 (codon 918).

Specifically, for codons 609, 611, 618, 620, 630, 634, and 768, oligonucleotides (M) having 7 types of missense mutations and the one wild type oligonucleotide (W) are spotted; for codon 804, oligonucleotides (M) having 6 types of missense mutations and one wild type oligonucleotide (W) are spotted; and for codon 918, the oligonucleotide (M) having the predominant mutation type and one wild type oligonucleotide (W) are spotted. To be more specific, used for codons 609, 611, 618, 620, 630, and 634 are 7 types of oligonucleotides obtained by replacing TGC (cysteine) with CGC (arginine), AGC (serine), GGC (glycine), TTC (phenylalanine), TAC (tyrosine), TCC (serine) and TGG (tryptophan), respectively. Used for codon 768 are 7 types of oligonucleotides obtained by replacing GAG (glutamic acid) with CAG (glutamine), AAG (lysine), GCG (alanine), GGG (glycine), GTG (valine), GAC (aspartic acid) and GAT (aspartic acid), respectively. For codon 804, 6 types of mutated oligonucleotides are used: GTG (valine) was replaced with CTG (leucine), ATG (methionine), TTG (leucine), GAG (glutamic acid), GCG (alanine) and GGG (glycine), respectively. Codon 918 mutant is oligonucleotide obtained by replacing ATG (methionine) with ACC (thereonine).

One wild type of oligonucleotide (W) is designed for each codon to be directly compared with mutation types. For example, 8 oligonucleotides are spotted for codon 618, one is to detect a normal base sequence and the rest, the mutated base sequences. As a whole, 56 mutant oligonucleotides are designed for the 56 missense mutation types at the 9 hot spot codons, and 11 oligonucleotides, for the wild types and positive controls (see Tables 1a and 1b). The positive controls are designed for exons 10 and 11 in which most mutations have been identified.

The three oligonucleotides described in SEQ ID Nos. 4, 20, and 44, respectively, are designed to have a shorter length of 16-mer rather than 20-mer. Preliminary experiments have revealed that some G-A mismatches often occur to exhibit non-specific signals when 20-mer oligonucleotides are used (positions 4, 20, and 44). For example, in case of the oligonucleotides described in SEQ ID Nos. 4 (-GGC-) and 8 (-TGC-), the complementary sequence (-ACG) in a sample DNA hybridizes only with the oligonucleotide of SEQ ID No. 8, in principle. However, it is possible to non-specifically hybridize with the oligonucleotide of SEQ ID No. 4 showing a constant level of binding affinity by G-A mismatch. It was reported that G-T and G-A mismatches slightly destabilize a duplex, while A-A, T-T, C-T, and C-A mismatches cause significant destabilization (Ikuta S. et al., *Nucleic Acids Res.* 15: 797-811, 1987). It was also reported that a high washing temperature enables a clear distinction between a perfectly matched duplex and a mismatched duplex (Ikuta S. et al., *Nucleic Acids Res*. 15: 797-811, 1987), but at room temperature ~ 60 °C , a sufficient distinction between specific signals and non-specific signals may not be possible. Thus, to reduce the experimental error caused by non-specific hybridization in the present invention, the length of the oligonucleotides is reduced from 20-mer to 16-mer in three cases (positions 4, 20, and 44) in order to decrease the destabilizing effect caused by G-A mismatch. These 16-mer oligonucleotides are analyzed with perfectly matched oligonucleotides.

The RET oligo chip of the present invention may be manufactured by fixing as many as 67 oligonucleotides on the surface of a solid matrix using an automatic microarrayer by a process comprising the steps of:
1) mixing each of the oligonucleotides in a micro spotting solution and distributing to a well plate;
2) spotting the oligonucleotide on the surface of a solid matrix using a microarrayer;
3) fixing the oligonucleotides on the solid matrix surface and washing;
4) denaturing the fixed oligonucleotides by soaking the solid matrix in 95°C water, and then, treating the solid matrix with a sodium borohydride solution; and
5) washing and drying the solid matrix.

Each of the oligonucleotides used in step (1) has preferably a functional group that can be used to form stable binding with the solid matrix surface. For example, each oligonucleotide may be linked with a 12 carbon spacer having a 5' amino function, e.g., H₂N-(CH₂)₁₂-oligonucleotide. This amine group undergoes Schiff's base reaction with an aldehyde group on the solid matrix to form a firm bond therebetween. The 12 carbon spacer serves to enhance the hybridization rate by facilitating the contact between the oligonucleotide and a fluorescent-labeled target DNA.

The micro spotting solution used in step (1) may contain suitable salts and polymers to facilitate the application of the oligonucleotides on the solid matrix.

The solid matrix used in step (2) may be made of a glass; a modified silicone; a plastic cassette; or a polymer such as polycarbonate or a gel thereof. The surface of a solid matrix may be coated with a chemical compound that can serve to bind the oligonucleotide to the matrix substrate. Preferable chemicals that can be used for such coating have functional groups such as aldehyde or amine groups. In one preferred embodiment, the present invention uses a slide glass coated with an aldehyde.

According to one embodiment of steps (1) and (2), a total of 268 oligonucleotides are arranged in a specified manner on a solid matrix using an automatic pin microarrayer. Each oligonucleotide spot is preferably of circular shape with a diameter ranging from 100 to 500 µm. A preferable example of the solid matrix is a slide glass having a size of about 3.7 cm × 7.6 cm, which can accommodate approximately 100 to 10,000 spots per chip. Preferably, a total of 268 oligonucleotide spots, each of 130 µm diameter, may be arranged in multiple columns and rows at intervals of 300 µm (see Fig. 2).

In step (3), the oligonucleotides are fixed on the solid matrix surface by way of forming covalent bonds between the amine groups of the oligonucleotide and the aldehyde groups of the solid matrix via Schiff's base reaction. Free unreacted oligonucleotides are removed by washing the solid matrix with SDS, SSC, SSPE, etc.

In step (4), the fixed oligonucleotides are denatured, and unreacted aldehyde groups remaining on the solid matrix are reduced and inactivated by sodium borohydride treatment.

The RET oligo chip of the present invention manufactured by the above process may be advantageously used to detect gene mutation and this inventive method is much simpler and more economical than any of the conventional gene mutation detection methods: It takes several days to months on the average when the presence of gene mutation is examined using such conventional methods as SSCP (single strand conformation polymorphism), PTT (protein truncation test), RFLP (restriction fragment length polymorphism), cloning, direct sequencing, etc. However, analysis of a DNA sample for RET gene mutation takes less than 9 hours when the inventive RET oligo chip is employed. In addition, the RET oligo chip of the present invention can be manufactured much more simply at a much less production cost than conventional chips. Once the required oligonucleotides are synthesized, it is possible to mass-produce the inventive slides. The amounts of reagents required when the inventive RET oligo chip is used are far less than those required in any of the conventional methods.

The RET oligo chip of the present invention is easy to manufacture using a pin microarrayer, while the existing Affymetrix oligo chip must be prepared using a complicated and expense phothlithography technique.

Further, it is pssible with the RET oligo chip of the present invention to purify and modify the oligonucleotides, in contrast to the case of Affymetrix oligo chip which is prepared by directly synthesizing oligonucleotides on the surface of a solid matrix, wherein it is not possible to purify or modify the oligonucleotides. Accordingly, the inventive RET oligo chip is capable of providing greater experimental accuracy than was possible before.

The present disclosure provides a method for detecting hereditary cancer employing the RET oligo chip, which comprises the steps of:
1) preparing a fluorescence-labeled DNA sample from the blood of a subject patient;
2) reacting the labeled DNA sample with oligonucleotide spots on the RET oligo chip;
3) washing the reacted oligo chip to remove unbound sample DNA;
4) detecting the mode of hybridization of specific oligonucleotide spots using a fluorescence reader; and
5) examining the presence of gene mutation.

In step (1), a DNA sample is prepared by incorporating a fluorescent dye into a blood DNA sample obtained from a subject patient. In the hybridization of fluorescent dye-labeled DNA with certain oligonucleotide spot on the oligo chip can be analyzed with a fluorescence reader using an appropriate software. Preferable fluorescent dyes include, but are not limited to, Cy5 and Cy3.

In step (2), the florescent dye-labeled DNA sample prepared in step (1) is mixed with a hybridization solution and transferred to each of the oligonucleotide. The hybridization reaction is performed in a 45~60°C incubator saturated with water vapor for 3~9 hours. Then, the oligo chip is washed to remove unbound sample DNA and dried (step 3), and the resulting fluorescence is analyzed with a fluorescence reader using an appropriate software (step 4). In step (5), setting a maximum value in 99% reliable range as a threshold value, any signal showing a fluorescence level higher than the threshold is regarded positive for the presence of mutation.

The RET oligo chip of the present invention can be effectively used to diagnose such hereditary cancer as MEN2A, MEN2B, FMTC, etc. Since RET gene belongs to the receptor tyrosine kinase family responsible for the cell signal transduction pathway, the inventive RET oligo chip can be used as an effective diagnostic tool for the study of cancer cells.

The following Examples and Test Examples are given for the purpose of illustration only, and are not intended to limit the scope of the invention.

### Example 1: Examination of RET mutation using RET oligo chip

### (Step 1) Manufacture of RET oligo chip

Sixty seven types of oligonucleotides, designed to detect all 56 missense mutation types at 9 mutational hot spot codons of RET gene, were manufactured as follows (Tables 1a and 1b). The oligonucleotides 10-PC and 11-PC described in SEQ ID Nos. 1 and 50 are positive controls, and the oligonucleotides described in SEQ ID Nos. 9, 17, 25, 33, 41, 49, 58, 65 and 67 are wild types. Other oligonucleotide each have missense mutation at one of the hot spot codons: the oligonucleotides described in SEQ ID Nos. 2 to 8, at codon 609; the oligonucleotides described in SEQ ID Nos. 10 to 16, at codon 611; the oligonucleotides described in SEQ ID Nos. 18 to 24, at codon 618; the oligonucleotides described in SEQ ID Nos. 26 to 32, at codon 620; the oligonucleotides described in SEQ ID Nos. 34 to 40, at codon 630; the oligonucleotides described in SEQ ID Nos. 42 to 48, at codon 634; the oligonucleotides described in SEQ ID Nos. 51 to 57, at codon 768; the oligonucleotides described in SEQ ID Nos. 59 to 64, at codon 804; and the oligonucleotide described in SEQ ID No. 66, at codon 918,

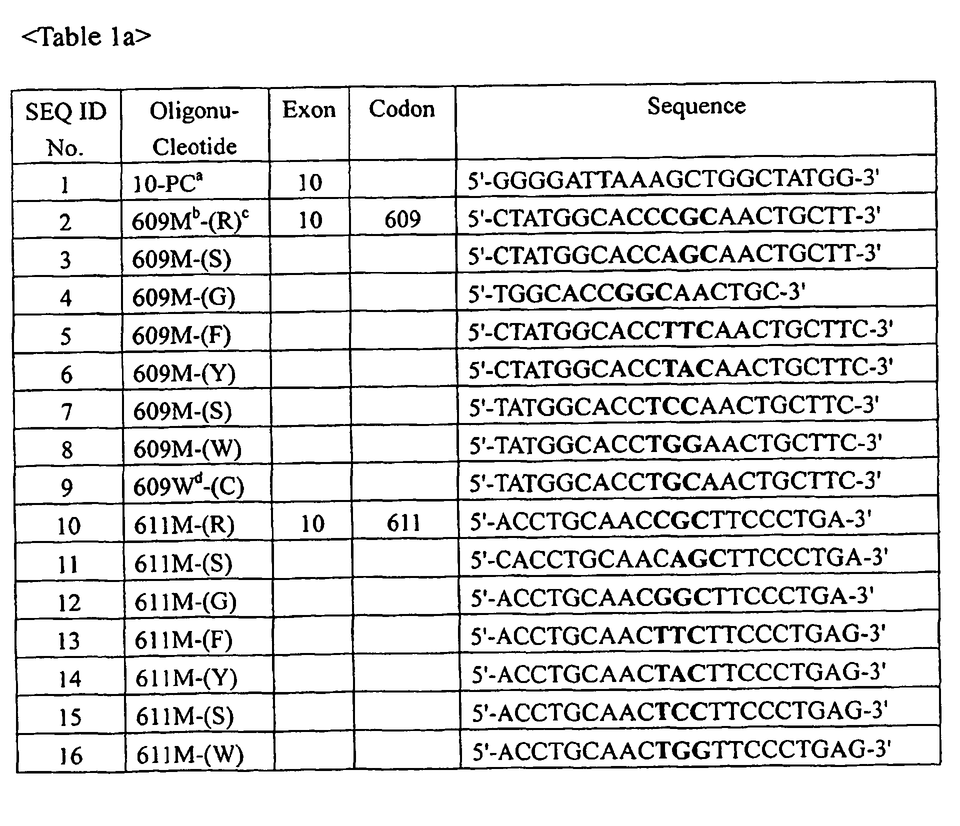

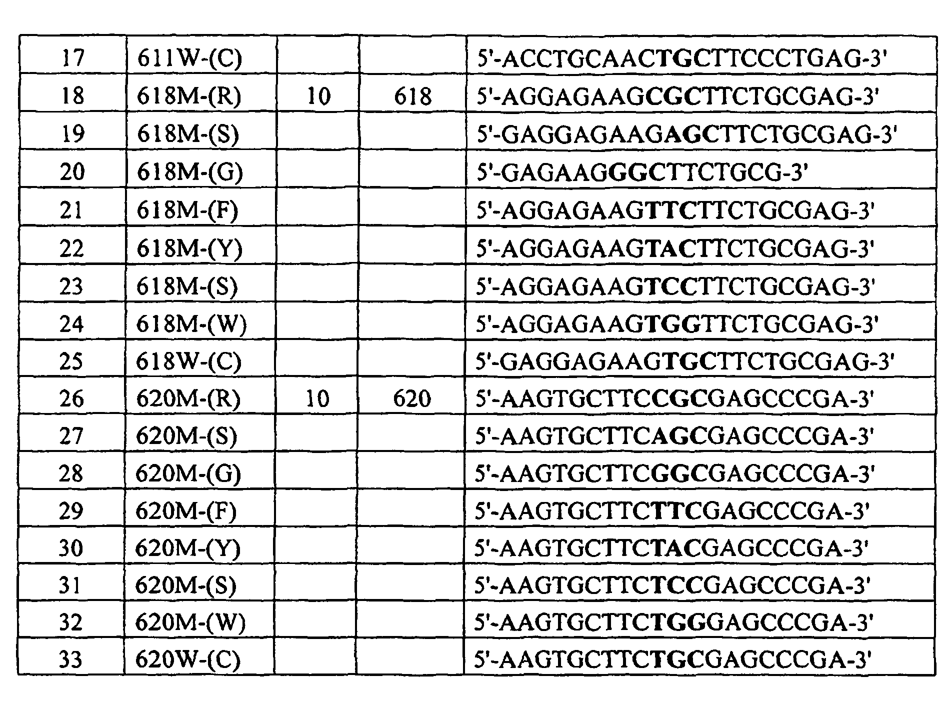

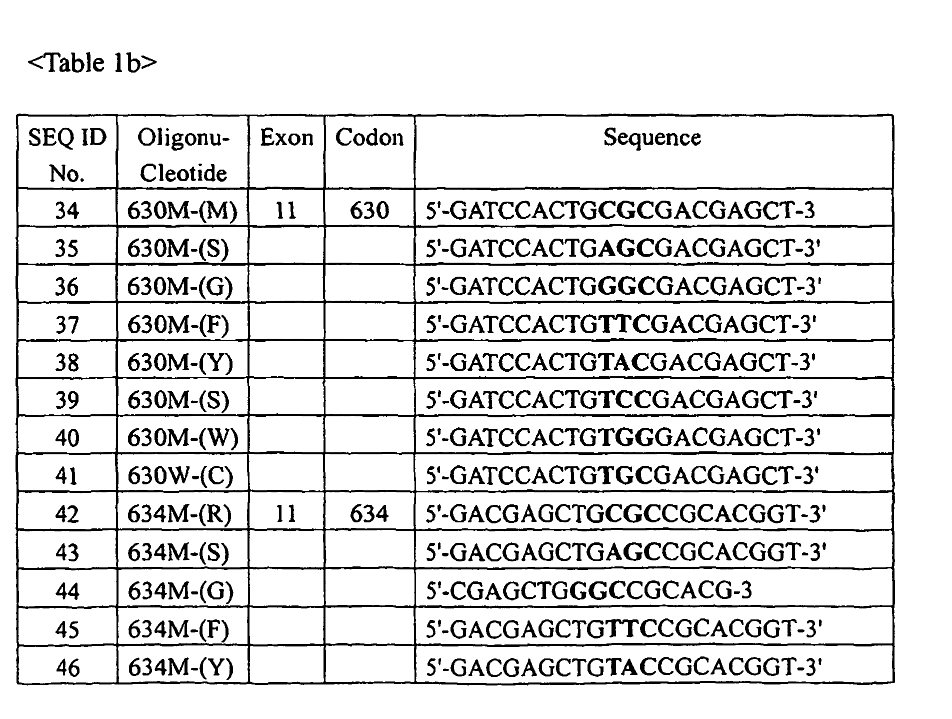

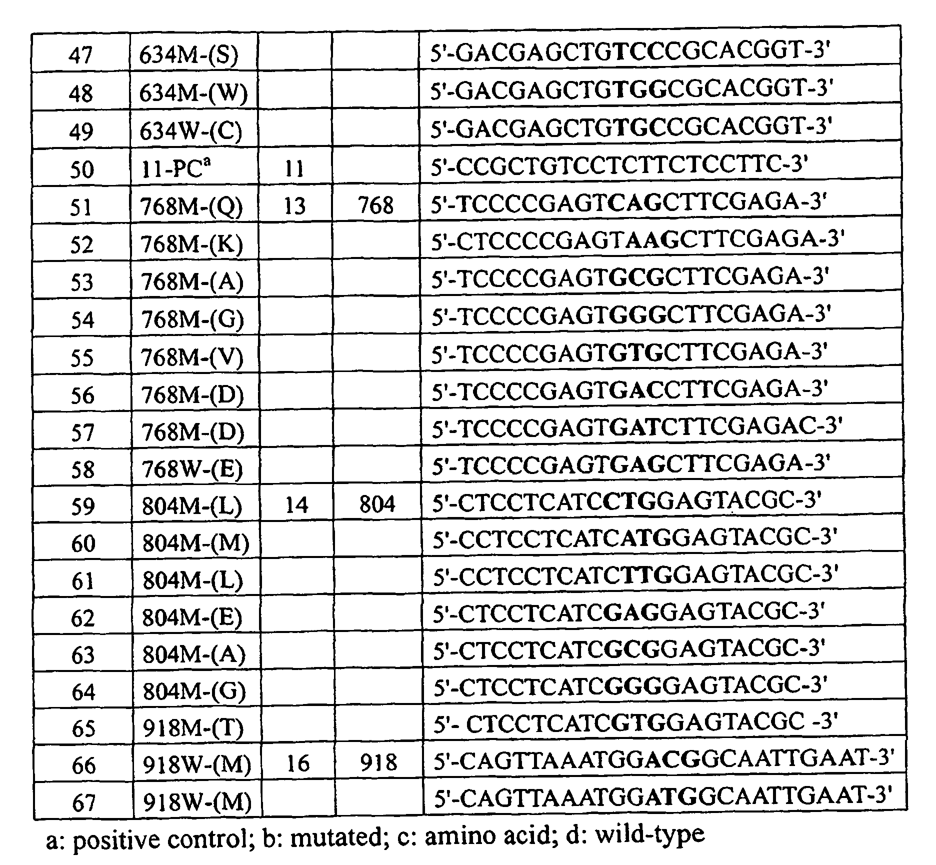

All 67 oligonucleotides, each having a 12 carbon spacer to 5'-terminal modified with an amine residue which can undergo Schiff's base reaction with aldehyde groups, were obtained from MWG-Biotech (Ebrsberg, Germany) and fixed on a glass slide. Twenty pmole/µℓ of each oligonucleotide was mixed with a micro spotting solution (TeleChem International Inc, Sunnyvale, CA) at a mix ratio of 1:1, resulting in 10 pmol/µℓ of final volume, and 40 µℓ of each oligonucleotide was transferred to a 96 well plate. After the charged 96 well plate was placed in a pin microarrayer (Microsys 5100 Cartesian, Cartesian Technologies Inc, Irvine, CA), each oligonucleotide was printed on an aldehyde-coated glass slide (26× 76× 1 mm, CEL Associates Inc, Houston, TX). Spots, each of 130 µm dameter in size, were arranged in multiple columns and rows at intervals of 300 µm. The glass slide spotted with the oligonucleotides was washed twice with 0.2% SDS and then, once with distilled water. The glass slide was soaked in hot water (95 °C) to denature the oligonucleotides, and then, in sodium borohydride solution for 5 minutes to inactivate unreacted aldehyde groups. Then, the glass slide was washed twice with 0.2% SDS, and then, once with distilled water, centrifuged, and dried.

### (Step 2) Preparation of DNA sample

Blood samples were collected from twenty three individuals related to five MEN2A families (Table 2). Twenty two of the 23 (SNU-MEN1A-I, II, III, IV) were confirmed to be MEN2A patients by a conventional method, and the remaining one (SNU-MEN2A-V) was an unknown subject.

**<Table 2>**

| Family | No. of affected members | No. of gene carriers | Mutation | Direct sequencing^{a} | Cloning and sequencing | RET oligo chip |
|---|---|---|---|---|---|---|
| I | 5 | 4 | C634W^{b} | +^{c} | + | + |
| II | 2 | 3 | C634R | + | + | + |
| III | 2 | 1 | C634R | + | + | + |
| IV | 11 | 9 | C618S | + | + | + |
| V | 1 | - | C634Y | No detection | + | + |

| | | | | | | |
|---|---|---|---|---|---|---|
| a: sequencing; | | | | | | |
| b: mutation at position of codon 634 (TGC→ TGG, Cys→ Trp); | | | | | | |
| c: detection | | | | | | |

In Table 2, the term of 'affected member' means a MEN2A patient showing clinical symptoms of MEN2A, and the term of 'gene carrier' means a mutated RET gene carrier not showing clinical symptoms of MEN2A.

Total genomic DNA was extracted from each blood sample using Ficoll-Paque (Amersham Pharmacia-biotech Ltd, Uppsala, Sweden) and TRI reagent (Molecular Research Center, Cincinati, OH), following the manufacture's instructions. To generate a fluorescent-labeled DNA sample, PCR amplification was performed using the extracted DNA as a template and five pairs of primers described in SEQ ID Nos. 68 to 77 (MWG-Biotech, Ebersberg, Germany) (Table 3). PCR primers for exon 10 and 11 were used as described in Takiguchi-Shirahama, S. et al., *Hum. Genet.* 95:187-190, 1995, PCR primers for exon 14 were used as described in Shuffenecker I. et al., *Am. J. Hum. Genet.* 62:233-237, 1997, PCR primers for exon 13 and 16 were used as described in Karaga, H. J. et al., *Eur. J. Endocrinol.* 139:410-415, 1998). PCR reaction solution was composed of 100 ng of genomic DNA, 10 pmol of each primer, 40 uM of dCTP, 20 uM of fluorescent dye Cy5-dCTP (MEN) or Cy3-dCTP (Amersham-biotech Ltd., Buckinghamshire, UK) in a volume of 25 µℓ. Reactions were initiated by denaturation for 5 min at 94°C in a programmable thermal cycler (Perkin Elmer Cetus 9600; Roche Molecular Systems, Inc., NJ). PCR conditions consisted of 35 cycles of 30 sec at 94°C, 30 sec at 60°C, and 1 min at 72°C, with a final elongation of 7 min at 72°C. Each exon was amplified separately. After the PCR amplification, Cy5- or Cy3-labeled PCR product was purified using a purification kit (Qiagen Inc, Valencia, CA) and digested with 0.25 U of DNase I (Takara, Shiga, Japan) at 25°C for 10 min. Remaining enzyme was inactivated at 95°C for 10 min and removed by the above purification procedure, the Cy5- or Cy3-labeled DNA sample was recovered.

**<Table 3>**

| SEQ ID No. | Primer | Amplifying region | Amplified size |
|---|---|---|---|
| 68 | Exon-10F | Exon 10 | 199 by |
| 69 | Exon-10R | | |
| 70 | Exon-11F | Exon 11 | 279 bp |
| 71 | Exon-11R | | |
| 72 | Exon-13F | Exon 13 | 209 bp |
| 73 | Exon-13R | | |
| 74 | Exon-14F | Exon 14 | 287 bp |
| 75 | Exon-14R | | |
| 76 | Exon-16F | Exon 16 | 186 bp |
| 77 | Exon-16R | | |

### (Step 3) Hybridization reaction and analysis

The Cy5- or Cy3-labeled DNA samples of each exon prepared in step (2) were each mixed and resuspended in pre-warmed 1× UniHyb solution (TeleChem International Inc, Sunnyvale, CA) to a volume of 2-4 µℓ. 2 µℓ of the mixed DNA sample was dropped on the glass slide prepared in step (1) and the glass slide was covered with a cover glass. The hybridization reaction was performed by incubating the glass slide in a saturated vapor tube at 60°C for 3 hours. The hybridized glass slide was rinsed at room temperature in a buffer of 2x SSC + 0.2% SDS for 10~30 min, and then, in distilled water for 5 min, followed by centrifuging and drying. The glass slide was scanned using a ScanArray Lite (Parkard Instrument Co, Meriden, CT) and analyzed using Quantitative Microarray analysis software (QuantArray, version 2.0). To establish a threshold value, all data analysis was carried out using a SigmaPlot (SPSS Inc., San Rafael, CA), and means and standard deviations were calculated.

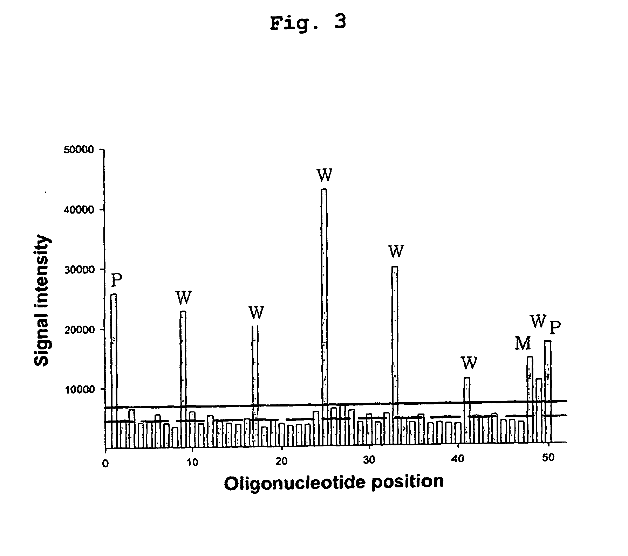

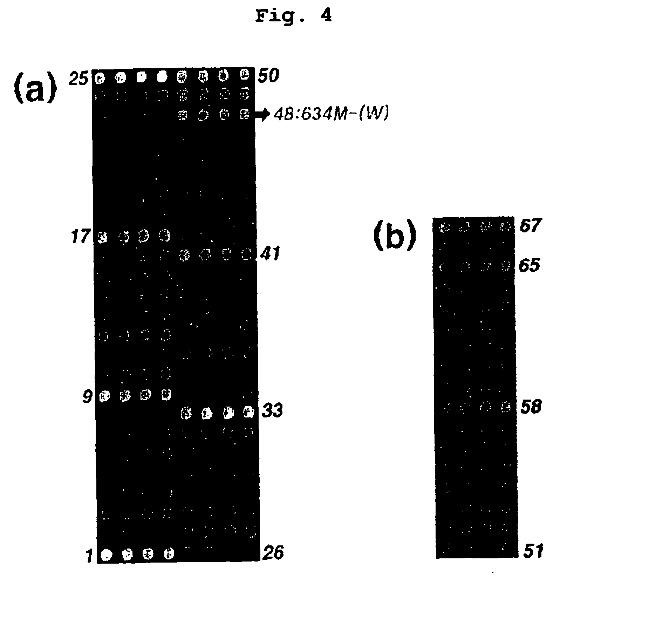

The positive signal for the presence of RET mutation on each codon was analyzed by setting the maximum value at the 99% reliable range as the threshold value (Figs. 3 and 4). Fig. 4 (a) shows the extents of hybridization occurred at exon 10 (codons 609, 611, 618, and 620) and exon 11 (codon 630 and 634). More than 95% of MEN2A mutations have been represented to occur at exon 10 and exon 11. Fig. 4 (b) indicates hybridization at exon 13 (codon 768) and 14 (codon 804). Most FMTC mutations have been identified at exon 13 and exon 14; while most MEN2B mutations have been observed occur at exon 16 (codon 918).

Hybridization results of 22 samples (SNU-MEN2A-I, II, III, IV) were consistent with the previous sequencing data. As to the unknown SNU-MEN2A-V sample, the present hybridization test with the inventive RET oligo chip revealed that a mutation from TGC (cysteine) to TGG (tryptophan) at codon 634 had taken place, instead of the wild type sequence determined previously using a conventional method. To confirm this mutation at codon 634, cloning and direct sequencing were performed. PCR product of exon 11 was ligated into PCR-TOPO vector, and subcloned using the TA cloning system (Invitrogen, Caelsbad, CA). Bi-directional sequencing was performed using a Taq dideoxy terminator cycle sequencing kit and an ABI 377 DNA sequencer (Perkin-Elmer, Foster City, CA). As a result, it was confirmed that SNU-MEN2A-V sample has a mutation (48:C634W, TGC→ TGG, exon 11) at codon 634. Namely, the RET oligo chip of the present invention can detect the mutation of RET gene missed by direct sequencing.

The features disclosed in the foregoing description, in the claims and/or the accompanying drawings may, both separately and in any combination thereof, be material for realising the invention in diverse terms thereof.

## Claims

1. A RET oligonucleotide microchip for detecting RET mutations which comprises oligonucleotides consisting of the nucleotide sequences SEQ ID Nos. 1 to 67 fixed on the surface of a solid matrix, said oligonucleotides being designed to detect missense mutation types hot spots of RET gene.

2. The RET oligonucleotide microchip of claim 1, wherein the hot spots are codons 609, 611, 618, 620, 630, 634, 768, 804 and 918.

3. The RET oligonucleotide microchip of claim 1, wherein the oligonucleotides are designed to detect 7 types of missense mutation at codons 609, 611, 618, 620, 630, 634, and 768, 6 types of missense mutation at codon 804, 1 type of missense mutation at codon 918, 9 types of wild type to each codon, and 2 types of positive control for exon 10 and 11.

4. The RET oligonucleotide microchip of claim 3, wherein the oligonucleotides described in SEQ ID Nos. 2 to 8 are for the detection of missense mutations at codon 609, SEQ ID Nos. 10 to 16 for missense mutations at codon 611, SEQ ID Nos. 18 to 24 for missense mutations at codon 618, SEQ ID Nos. 26 to 32 for missense mutations at codon 620, SEQ ID Nos. 34 to 40 for missense mutations at codon 630, SEQ ID Nos. 42 to 48 for missense mutations at codon 634, SEQ ID Nos. 51 to 57 for missense mutations at codon 768, SEQ ID Nos. 59 to 64 for missense mutations at codon 804, and SEQ ID No. 66 for missense mutations at codon 918.

5. A manufacturing process of the RET oligonucleotide microchip of any of claims 1 to 5, comprising
1) mixing each of the oligonucleotides in a micro spotting solution and distributing to a well plate, wherein the oligonucleotides consist of the nucleotide sequences of SEQ ID Nos. 1 to 67;
2) spotting the oligonucleotides on the surface of a solid matrix using a microarrayer;
3) fixing the oligonucleotides on the solid matrix surface and washing;
4) denaturing the fixed oligonucleotides by soaking the solid matrix in 95°C water, and then, treating the solid matrix with a sodium borohydride solution; and
5) washing and drying the solid matrix.

6. The manufacturing process of claim 5, wherein each of the oligonucleotides used in step (1) has a 12 carbon spacer with 5' amino modification.

## Patentansprüche

1. Ein RET-Oligonukleotid-Microchip zum Nachweis von RET-Mutationen, der Oligonukleotide umfaßt, die aus den Nukleotidsequenzen der SEQ ID Nm. 1 bis 67 bestehen, die auf die Oberfläche einer festen Matrix fixiert sind, wobei die Oligonukleotide so aufgebaut sind, um Missense-Mutationstypen Hot-Spots des RET-Gens nachzuweisen.

2. RET-Oligonukleotid-Microchip nach Anspruch 1, wobei die Hot-Spots Codons 609, 611, 618, 620, 630, 634, 768, 804 und 918 sind.

3. RET-Oligonukleotid-Microchip nach Anspruch 1, wobei die Oligonukleotide so aufgebaut sind, um 7 Typen von Missense-Mutation an Codons 609, 611, 618, 620, 630, 634 und 768, 6 Typen von Missense-Mutation an Codon 804, 1 Typ von Missense-Mutation an Codon 918, 9 Typen von Wild-Typ an jedem Codon und 2 Typen von Positiv-Kontrolle für Exon 10 und 11 nachzuweisen.

4. RET-Oligonukleotid-Microchip nach Anspruch 3, wobei die in SEQ ID Nm. 2 bis 8 beschriebenen Oligonukleotide zum Nachweis von Missense-Mutationen an Codon 609 vorgesehen sind, SEQ ID Nm. 10 bis 16 für Missense-Mutationen an Codon 611, SEQ ID Nm. 18 bis 24 für Missense-Mutationen an Codon 618, SEQ ID Nm. 26 bis 32 für Missense-Mutationen an Codon 620, SEQ ID Nm. 34 bis 40 für Missense-Mutationen an Codon 630, SEQ ID Nm. 42 bis 48 für Missense-Mutationen an Codon 634, SEQ ID Nm. 51 bis 57 für Missense-Mutationen an Codon 768, SEQ ID Nm. 59 bis 64 für Missense-Mutationen an Codon 804 und SEQ ID Nr. 66 für Missense-Mutationen an Codon 918.

5. Herstellungsverfahren für den RET-Oligonukleotid-Mikrochip nach einem der Ansprüche 1 bis 5, umfassend
1) Mischen jedes der Oligonukleotide in einer Mikro-Spotting-Lösung und Verteilen auf einer Loch-Platte, wobei die Oligonukleotide aus den Nukleotidsequenzen von SEQ ID Nm. 1 bis 67 bestehen;
2) Spotten der Oligonukleotide auf die Oberfläche einer festen Matrix, unter der Verwendung eines Microarrayers;
3) Fixieren der Oligonukleotide auf der festen Matrixoberfläche und Waschen;
4) Denaturieren der fixierten Oligonukleotide durch Eintauchen der festen Matrix in 95°C Wasser und dann Behandeln der festen Matrix mit einer Natriumborhydridlösung; und
5) Waschen und Trocknen der festen Matrix.

6. Herstellungsverfahren nach Anspruch 5, wobei jedes der in Schritt (1) verwendeten Oligonukleotide einen 12 Kohlenstoff-Abstandhalter mit 5'-Aminomodifikation aufweist.

## Revendications

1. Micropuce RET à oligonucléotides pour détecter des mutations RET qui comprend des nucléotides consistant en les séquences de nucléotides SEQ ID Nos. 1 à 67 fixées sur la surface d'une matrice solide, lesdites nucléotides étant conçues pour détecter les points chauds des types de mutations faux-sens du gène RET.

2. Micropuce RET à oligonucléotides selon la revendication 1, dans laquelle les points chauds sont les codons 609, 611, 618, 620, 630, 634, 768, 804 et 918.

3. Micropuce RET à oligonucléotides selon la revendication 1, dans laquelle les oligonucléotides sont conçues pour détecter 7 types de mutations faux-sens sur les codons 609, 611, 618, 620, 630, 634 et 768, 6 types de mutations faux-sens sur le codon 804, 1 type de mutations faux-sens sur le codon 918, 9 types de type sauvage sur chacun des codons, et 2 types de régulation positive sur l'exon 10 et 11.

4. Micropuce RET à oligonucléotides selon la revendication 3, dans laquelle les oligonucléotides décrits dans les SEQ ID Nos. 2 à 8 sont pour la détection de mutations faux-sens sur le codon 609, dans les SEQ ID Nos. 10 à 16 pour les mutations faux-sens sur le codon 611, dans les SEQ ID Nos. 18 à 24 pour les mutations faux-sens sur le codon 618, dans les SEQ ID Nos. 26 à 32 pour les mutations faux-sens sur le codon 620, dans les SEQ ID Nos. 34 à 40 pour les mutations faux-sens sur le codon 630, dans les SEQ ID Nos. 42 à 48 pour les mutations faux-sens sur le codon 634, dans les SEQ ID Nos. 51 à 57 pour les mutations faux-sens sur le codon 768, dans les SEQ ID Nos. 59 à 64 pour les mutations faux-sens sur le codon 804, et dans la SEQ ID No. 66 pour les mutations faux-sens sur le codon 918.

5. Processus de fabrication d'une micropuce RET à oligonucléotides selon l'une quelconque des revendications 1 à 5, comprenant
1) le mélange de chacun des oligonucléotides dans une solution à microinjecter et la distribution sur une microplaque, dans lequel les nucléotides consistent en les séquences de nucléotides SEQ ID Nos. 1 à 67 ;
2) l'application par spots des oligonucléotides sur la surface d'une matrice solide en utilisant un microinjecteur ;
3) la fixation des oligonucléotides sur la surface de la matrice solide et le lavage ;
4) la dénaturation des oligonucléotides fixés en trempant la matrice solide dans une eau à 95°C, et ensuite, le traitement de la matrice solide avec une solution de borohydrure de sodium ; et
5) le lavage et le séchage de la matrice solide.

6. Processus de fabrication selon la revendication 5, dans lequel chacun des oligonucléotides utilisés dans l'étape (1) a un espaceur à 12 carbones avec une modification 5' amino.
